**Europäisches Patentamt**

(19) **European Patent Office**

**Office européen des brevets**

(11) Numéro de publication: **0 191 028**
**B1**

(12) # FASCICULE DE BREVET EUROPÉEN

(45) Date de publication du fascicule du brevet:
**12.07.89**

(51) Int. Cl.⁴: **C 07 K 7/00,** C 07 K 7/06,
A 61 K 37/02, G 01 N 33/569

(21) Numéro de dépôt: **85903301.1**

(22) Date de dépôt: **02.07.85**

(86) Numéro de dépôt international:
**PCT/FR 85/00183**

(87) Numéro de publication internationale:
**WO 86/00620 (30.01.86 Gazette 86/03)**

(54) **MOLECULE COMPORTANT AU MOINS UNE SEQUENCE PEPTIDIQUE PORTEUSE D'UN EPITOPE.**

(30) Priorité: **02.07.84 FR 8410461**

(43) Date de publication de la demande:
**20.08.86 Bulletin 86/34**

(45) Mention de la délivrance du brevet:
**12.07.89 Bulletin 89/28**

(84) Etats contractants désignés:
**AT BE CH DE GB IT LI LU NL SE**

(56) Documents cité:
**US-A-4 442 031**

(73) Titulaire: **INSTITUT PASTEUR, 25/28, rue du Docteur Roux, F-75015 Paris (FR)**
Titulaire: **CENTRE NATIONAL DE LA RECHERCHE SCIENTIFIQUE (CNRS), 15, Quai Anatole France, F-75007 Paris (FR)**

(72) Inventeur: **KOENEN, Michael, Wilhelmsfelder str. 16, D-6900 Heidelberg- Ziegelhsn (DE)**
Inventeur: **SCHERF, Arthur, Weyertal 20, D-5 Köln 41 (DE)**
Inventeur: **MÜLLER- HILL, Benno, Händelstr. 53, D-5 Köln 1 (DE)**
Inventeur: **MERCEREAU- PUIJALON, Odile, 11, bld. Voltaire, F-92130 Issy- les- Moulineaux (FR)**
Inventeur: **PEREIRA DA SILVA, Luiz, 10, square Port- Royal, F-75013 Paris (FR)**

(74) Mandataire: **Gutmann, Ernest, S.C. Ernest Gutmann - Yves Plasseraud 67, boulevard Haussmann, F-75008 Paris (FR)**

EP 0 191 028 B1

**Description**

L'invention concerne des molécules comportant une ou plusieurs séquences peptidiques porteuses d'un épitope caractéristique d'une protéine produite par des cellules infectées par le parasite du paludisme. Elle concerne également des compositions les contenant, plus particulièrement des composantes de vaccin contre le paludisme dont elles peuvent constituer l'un des principes actifs.

On sait que de nombreuses études ont été entreprises pour isoler des fractions polypeptidiques susceptibles de posséder des propriétés vaccinantes contre le paludisme à partir de souches purifiées de parasites dont a été reconnue la capacité d'infecter un hôte humain, telles que les souches connues sous les désignations Plasmodium falciparum ou Plasmodium knowlesi ou Plasmodium vivax pour ne citer que les principaux d'entre eux, ou à partir de cellules infectées, notamment d'érythrocytes infectés à des stades divers de l'évolution de la maladie. Plus récemment des banques de cADN clonés derivés des génomes de sporozoïtes de Plasmodium knowlesi et des formes sanguines (dont les mérozoites) de Plasmodium falcioarum ont été établies respectivement par Ellis J. et coll. (1983), Nature 302, 536 - 538 et Coppel, R.L. et coll. (1983), Nature, 306, 751 - 756. Il a été reconnu que ces banques comportaient des clones susceptibles d'exprimer des polypeptides contenant des unités répétitives de 12 (P. knowlesi) et de 11 (P. falciparum) aminoacides respectivement.

L'invention concerne plus particulièrement une autre catégorie de molécules caractérisées par la présence dans leur structure de une ou plusieurs séquences peptidiques caractéristiques d'une protéine résultant de l'activité infectieuse des susdits parasites dans les cellules, notamment érythrocytes, cette protéine étant au moins à certains stades de leur développement.

L'invention concerne tout d'abord un polypeptide, celui-ci étant formé, soit d'un monomère synthétique monapeptidique répondant respectivement à l'une des formules indiquées ci-dessous, soit d'un oligomère de l'un ou de plusieurs des monomères dont les formules sont indiquées ci-dessous:

| 1. | Glu | Glu | Val | Val | Glu | Glu | Val | Val | Pro |
|----|-----|-----|-----|-----|-----|-----|-----|-----|-----|
| 2. | ... | ... | Leu | ... | ... | ... | ... | ... | ... |
| 3. | Val | ... | Leu | ... | ... | ... | ... | ... | ... |
| 5. | ... | ... | Leu | ... | ... | ... | ... | Ile | ... |
| 6. | ... | ... | Ile | ... | ... | ... | ... | Ile | ... |
| 9. | ... | ... | Leu | Ile | ... | ... | ... | ... | ... |
| 10. | Val | ... | Leu | Leu | ... | ... | ... | Ile | ... |
| 11. | Val | ... | ... | Leu | ... | Asp | ... | Ile | ... |
| 13. | ... | ... | Ile | Ile | ... | ... | ... | Ile | ... |
| 14. | ... | ... | Ile | Ile | ... | ... | Ala | Ile | ... |
| 15. | Val | ... | Ile | ... | ... | ... | ... | Ile | ... |
| 16. | ... | Asp | Ile | ... | ... | ... | ... | Ile | ... |
| 17. | ... | ... | Ile | ... | ... | Asp | ... | Ile | ... |
| 20. | ... | ... | Leu | ... | ... | ... | ... | Ile | ... |
| 22. | ... | ... | ... | Pro | ... | ... | ... | ... | ... |
| 23. | Glu | Glu | Leu | ... | ... | ... | ... | ... | ... |

étant entendu que dans chacune des séquences nonapeptidiques ci-dessous les groupes des trois points respectivement situés à la verticale des résidus aminoacyle de la première des séquences nonapeptidiques susmentionnées sont identiques à ces aminoacyles et que, dans ces monomères:

- chacun des acides aminés isoleucine, leucine et valine peut, dans ces séquences, être remplacé par l'un des deux autres, et que
- Glu peut être remplacé par Asp ou Gln,
- Val peut être remplacé par Ala ou Gly,
- Leu peut être remplacé par Ala.

Il va de soi que les fonctions réactives libres que sont susceptibles de posséder certains résidus aminoacides entrant dans la constitution des molécules selon l'invention, notamment les groupes carboxyles libres portés par les groupes Glu, peuvent être modifiées, dès lors que cette modification n'entraîne pas une modification des propriétés antigéniques, le cas échéant immunogènes de l'ensemble de la molécule. Les molécules ainsi modifiées entrent naturellement dans le cadre de la protection donnée à l'invention par les revendications. Ces fonctions carboxyles sont éventuellement acylées ou estérifiées.

Les peptides selon l'invention peuvent être préparés par les techniques classiques, dans le domaine de la synthèse des peptides. Cette synthèse peut être réalisée en solution homogène ou en phase solide.

Par exemple on aura recours à la technique de synthèse en solution homogène décrit par HOUBENWEYL dans l'ouvrage intitulé "Methode der Organischen Chemie" (Méthode de la chimieorganique) édité par E. Wünsch, vol. 15-I et II. THIEME, Stuttgart 1974.

Cette méthode de synthèse consiste à condenser successivement deux à deux les aminoacyles successifs dans l'ordre requis, ou à condenser des aminoacyles et des fragments préalablement formés et contenant déjà plusieurs résidus aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amine de l'un et carboxyle de l'autre ou vice versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-éthyl-3-(3-diméthyl-aminopropyl)-carbodiimide. Lorsque l'aminoacyle mis en oeuvre possède une fonction acide sipplémentaire (notamment dans le cas de l'acide glutamique), ces fonctions seront par exemple protégées, par des groupes t-butylester.

Dans le cas de la synthèse progressive, acide aminé par acide aminé, la synthèse débute de préférence par la condensation de l'amino-acide C-terminal avec l'aminoacide qui correspond à l'aminoacyle voisin dans la séquence désirée et ainsi de suite, de proche en proche, jusqu'à l'acide aminé N-terminal. Selon une autre technique préférée de l'invention, on a recours à celle décrite par R.D. MERRIFIELD dans l'article intitulé "Solid phase peptide synthesis" (J. Am. Chem. Soc., 45, 2149 - 2154).

Pour fabriquer une chaîne peptidique selon le procédé de MERRIFIELD, on a recours à une résine polymère très poreuse, sur laquelle on fixe le premier amino-acide C-terminal de la chaîne. Cet amino-acide est fixé sur la résine par l'intermédiaire de son groupe carboxylique et sa fonction amine est protégée, par exemple par le groupe t-butyloxycarbonyle.

Lorsque le premier amino-acide C-terminal est ainsi fixé sur la résine, on enlève le groupe protecteur de la fonction amine en lavant la résine avec un acide.

Dans le cas où le groupe protecteur de la fonction amine est le groupe t-butyloxycarbonyle, il peut être éliminé par traitement de la résine à l'aide d'acide trifluoroacétique.

On couple ensuite le deuxième amino-acide qui fournit le second amino-acyle de la séquence recerchée, à partir du résidu amino-acyle C-terminal sur la fonction amine déprotégée du premier amino-acide C-terminal fixé sur la chaîne. De préférence, la fonction carboxyle de ce deuxième amino-acide est activée, par exempel par la dicyclohexylcarbodiimide, et la fonction amine est protégée, par exemple par le t-butyloxycarbonyle.

On obtient ainsi la première partie de la chaîne peptidique recherchée, qui comporte deux aminoacides, et dont la fonction amine terminale est protégée. Comme précédemment, on déprotège la fonction amine et on peut ensuite procéder à la fixation du troisième aminoacyle, dans les conditions analogues à celles de on l'addition du deuxième aminoacide C-terminal.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents aminoacides constituant la chaîne peptidique et on détache le peptide de la résine par exemple à l'aide d'acide fluorhydrique.

L'invention concerne également les oligomères hydrosolubles des peptides monomères sus-indiqués. L'oligomérisation peut provoquer un accroîssement de l'immunogénicité des peptides monomères selon l'invention. Sans qu'une telle indication chiffrée puisse être considérée comme limitative, on mentionnera néanmoins que ces oligomères peuvent éventuellement contenir de 2 à 25 unités monomères.

On peut avoir recours, pour réaliser l'oligomérisation, à toute technique de polymérisation couramment utilisée dans le domaine des peptides, cette polymérisation étant ocnduite jusqu'à l'obteniton d'un oligomère ou polymère contenant le nombre de motifs monomères requis pour l'acquisition de l'immunogénicité désirée.

Une méthode préférée de l'oligomérisation ou de polymérisation du monomère consiste dans la réaction de celui-ci avec un agent de réticulation tel que le glutaraldéhyde.

On peut également avoir recours à d'autres méthodes d'oligomérisation ou de couplage, par exemple à celle mettant en jeu des couplages successifs d'unités monomères, par l'intermédiaire de leurs fonctions terminales carboxyle et amine en présence d'agents de couplage homo- ou hétéro- bifonctionnels.

On peut également pour la production de molécules comportant un ou plusieurs motifs nonapeptidiques tels que définis ci-dessus, avoir recours à des techniques du génie génétique mettant en oeuvre des microorganismes transformés par un acide nucléique déterminé comprenant des séquences nucléotidiques appropriées correspondantes. Parmi les séquences nucléotidiques correspondantes on mentionnera celles qui sont caractérisées par les séquences de triplets qui suivent, ces séquences correspondant en particulier aux peptides a) à g) dont les formules ont été indiquées (étant entendu que les nucléotides qui sont remplacés par des points dans les sèquences 2 à 23 ci-après sont identiques à ceux de la première séquence à l'aplomb duquel ils se trouvent).

```
1. GAA GAA GTT  GTT  GAA GAA GTT  GTG CCT
2. ...    ...   T.A  ...  ...  ...  ...  ..A  ..A
3. .T.    ...   C.A  ..A  ...  ...  ...  ..A  ...
4. .T.    ...   C.A  ..A  ...  ...  ...  ..A  ..A
5. ..G    ...   C.A  ...  ...  ..G  ...  A.A  ...
6. ...    ...   A..  ..G  ...  ...  ...  A.A  ...
7. ...    ...   ..C  ...  ...  ...  ..A  ..A  ...
8. ..G    ...   A.A  ...  ...  ...  ..G  A.A  ...
```

```
 9. ...   ...   T.A  A..  ...   ...   ...   ...   ..A
10. .T.   ...   C.A  T.A  ...   ...   ...   A.A   ..A
11. .T.   ...   ..A  T.A  ...   ..T   ...   A.A   ...
12. ...   ...   ...  ...  ...   ...   ...   A.A   ..A
13. ...   ...   A..  A..  ...   ...   ...   A.A   ...
14. ...   ...   A..  A..  ...   ..G  .CG    A.A   ..A
15. .T.   ...   A..  ...  ...   ..G   ...   A.A   ..A
16. ...   ..T   A..  ...  ...   ...   ...   A.A   ..A
17. ...   ...   A..  ...  ...   ..T   ...   A.A   ..A
18. ...   ...   ...  ...  ...   ...   ...   A.A   ...
19. ...   ...   ...  ...  ...   ...   ...   ..A   ..A
20. ...   ...   T.A  ..A  ...   ...   ...   A.A   ..A
21. ...   ...   C.A  ...  ...   ..G  ..G    A.A   ...
22. ...   ...   ...  CC.  ...   ..G   ...   ..A   ..A
23. GAA GAA CTC ...   ...   ...   ...   ...   ...
```

A ce titre l'invention concerne également des acides nucléiques contenant une ou plusieurs de ces séquences répétitives comprenant chacune 9 triplets du genre sus-indiqué. Elle concerne plus particulièrement des fragments d'acide nucléique comportant au plus 1000 paires de bases et l à 25 de ces séquences répétitives de 9 triplets chacune.

L'invention concerne encore les conjugués obtenus par couplage covalent des peptides selon l'invention (ou des susdits oligomères) à des molécules porteuses (naturelles ou synthétiques), physiologiquement acceptables et non toxiques, par l'intermédiaire de groupements réactifs complémentaires respectivement portés par la molécule porteuse et le peptide. Des exemples de groupements appropriés sont illustrés dans ce qui suit:

A titre d'exemples de molécules porteuses ou supports macromoléculaires entrant dans la constitution des conjugués selon l'invention, on mentionnera des protéines naturelles, telles que l'anatoxine tétanique, l'ovalbumine, des sérums albumines, des hémocyamines, etc...

A titre de supports macromoléculaires synthétiques, on mentionnera par exemple des polylysines ou des poly (D-L-alanine)-poly(L-lysine).

La littérature mentionne d'autres types de supports macromoléculaires susceptibles d'être utilisés, lesquels présentent en général un poids moléculaire supérieur à 20 000.

Pour synthétiser les conjuqués selon l'invention, on peut avoir recours à des procédés connus en soi, tels que celui décrit par FRANTZ et ROBERTSON dans Infect. and Immunity, <u>33</u>, 193 - 198 (1981), ou celui décrit dans Applied and Environmental Microbiology, octobre 1981, vol. 42, n° 4, 611 - 614 par P. E. KAUFFMAN en utilisant le peptide et la molécule porteuse appropriée.

Dans la pratique, on utilisera avantageusement comme agent de couplage les composés suivants, cités à titre non limitatif: aldéhyde glutarique, chloroformiate d'éthyle, carbodiimides hydrosolubles [N-éthyl-N'(3-diméthylamino-propyl) carbodiimide, HC17], diisocyanates, bis-diazobenzidine, di- et trichloro-s-triazines, bromures de cyanogène, benzoquinone, ainsi que les agents de couplage mentionnes dans Scand. J. Immunol., 1978, vol. 8, p. 7 - 23 (AVRAMEAS, TERNYNCK, GUESDON).

On peut avoir recours à tout procédé de couplage faisant intervenir d'une part une ou plusieurs fonctions réactives du peptide et d'autre part, une ou plusieurs fonctions réactives des molecules supports. Avantageusement, il s'agit des fonctions carboxyle et amine, lesquelles peuvent donner lieu à une réaction de couplage en présence d'un agent de couplage du genre de ceux utilisés dans la synthèse des protéines, par exemple, le 1-éthyl-3-(3-diméthylaminoprooyl)-carbodiimide, le N-hydroxybenzotriazole, etc. On peut encore avoir recours à la glutaraldéhyde, notamment lorsqu'il s'agit de relier entre eux des groupes aminés respectivement portés par le peptide et la molécule support.

Des caractéristique supplémentaires de l'invention apparaîtront encore au cours de la description qui suit des conditions dans lesquelles un polypeptide contenant une pluralité des séquences nonapeptidiques dont la constitution a été indiquée, a été obtenu. Il sera fait référence dans ce oui suit aux dessins dans lesquels:

- la figure 1 représente de façon schématique la structure d'un plasmide modifié qui a été mis en oeuvre dans la production d'un polypeptide contenant les susdites séquences non apeptidiques et

- la figure 2 fournit la structure de parties d'un acide nucléque capable de produire le susdit polypeptide et dont la séquence a été réalisée en mettant en oeuvre la technique classique de Maxam et Gilbert (1980) Methods in Enzymology <u>65</u>, 499 - 560.

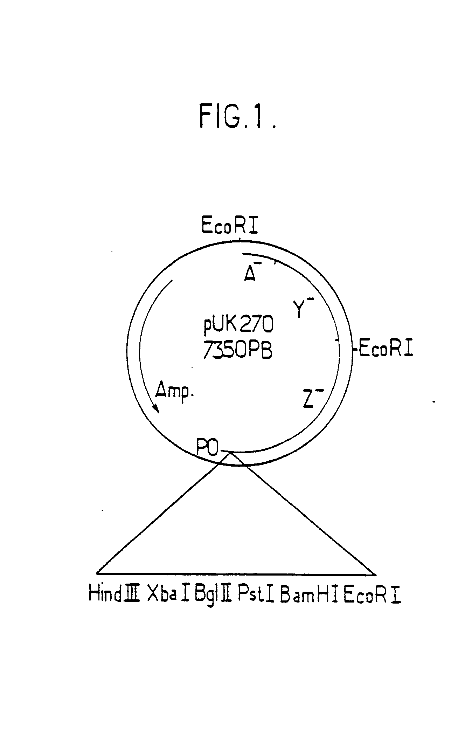

L'ADN génomique préalablement isolé à partir de la souche de <u>Plasmodium falciparum</u> (déposée à la Collection Nationale des Cultures de Microorganismes de l'Institut Pasteur de Paris sous le N° FUPC 1-212 le 23 décembre 1982) a été coupé par une DNAse en fragments ayant en général de l'ordre de 200 à 1000 paires de bases. Ces fragments ont été introduits dans le vecteur pUK 270, dont la structure résulte du schéma de la fig. 1 et qui est un dérivé du plasmide pUK 230 décit par M. KOENEN et coll. (1982) EMBO, 1, 509 - 512.

Ce vecteur comprend un operon-<u>lac</u> et, dans la partie non essentielle d'extrémité 5' du gène <u>lac</u> Z, une séquence nucléotidique de synthèse contenant la séquence des sites de restriction qui apparaît dans la fig. 1.

Le plasmide pUK 230 diffère du pUK 270 par la séquence polylinker insérée à la partie 5′ du gène codant par la β-galactosidase. Cette séquence nucléotidique de synthèse (polylinker) introduit dans le gène Z un décalage de phase dont l'effet est de rendre les gènes Z, Y et A, phénotypiquement Z-, Y- et A-.

L'introduction des fragments d'ADN génomique de Plasmodium falciparum dans ce vecteur a été réalisée dans le site Pst I, après ouverture de celui-ci et formation de brins monocaténaires oligo-G à ses extrémités. Les brins d'ADN avaient eux-mêmes été au préalable munis d'extrémités oligo-C, lesquelles ont alors permis la ligation de ces brins d'ADN dans le vecteur. L'introduction de ces fragments d'ADN dans les sites Pst I dans les conditions indiquées aboutit alors à des clones qui redeviennent lactose-positifs grâce à la remise en phase du gène Z avec son promoteur, dans les conditions également décrites Par M. KOENEN. Les clones sont repérés par la coloration bleue qu'ils donnent sur des plaques indicatrices contenant de la 5-bromo-4-chloro-3-indolyl-β-D-galactosidase (X-gal) ou l'isopropyl-1-thio-β-D-galactoside (JPTG) ou en tant que colonies lactose positives sur de l'agar-lactose. Au contraire les vecteurs ne contenant pas de fragments conduisent à des colorations bleu pâle sur les mêmes plaques indicatrices et sont caractérisés par un phénotype lactose-négatif. Les recombinants lactose-positifs expriment alors l'ADN inséré en tant que partie N-terminale d'une protéine chimère contenant l'essentiel de la β - galactosidase.

Les clones producteurs d'antigènes ont été identifiés par la méthode immuno-enzymatique décrite par M. KOENEN et coll. 10 000 clones rendus Lac+ ont ainsi été isolés et triés au moyen de divers anti-sérums dans les conditions qui seront rapportées ci-après.

On a tout d'abord utilisé des anticorps de lapins qui avaient été immunisés avec des mérozoïtes de Plasmodium falciparum dénaturées par le détergent connu sous la désignation TRITON x 100. Les sérums ont été prélevés sur les lapins après que ceux-ci eurent reçu 2 rappels. La fraction IgG a été isolée et mise en oeuvre dans la détection immuno-enzymatique.

Un deuxième tri a été réalisé en mettant en oeuvre un mélange d'anti-sérums obtenu à partir de 4 personnes qui possédaient un fort titre en anticorps dirigé contre Plasmodium falciparum et qui étaient originaires d'une zone endémique. Avant usage les anti-sérums ont été purifiés de tout anticorps dirigé contre E. coli par mise en contact avec des extraits de E. coli broyés, et dilués au 1/25 ème.

Les essais ont été réalisés plus particulièrement comme suit.

Les clones Lac+ ont été mis en culture sur de l'agar contenant du lactose en boîtes de Petri en verre et lysés dans les conditions décrites par M. KOENEN. Dans certains cas les cultures ont été réalisées sur des filtres en nitro-cellulose placés sur l'agar contenant le lactose. Les colonies qui s'étaient développées ont été lysées par contact, notamment des filtres, avec du chloroforme pendant 1 minute à la température ambiante. Le chloroforme á été éliminé par exposition des filtres à l'air pendant 5 minutes à 37°C. Les filtres ont ensuite été mis en contact avec des feuilles de chlorure de polyvinyle qui avaient au préalable été revêtues avec des anticorps selon la technique de BROOME S. et coll (1978) Proc. Natl. Acad. Sci. USA 75, 2746 - 2749 et saturées avec de la sérum-albumine dissoute dans la solution de lavage, telle que décrite par M. KOENEN et coll., cette solution contenant en outre le détergent TRITON x 100 en concentration de 0,5 % (vol/vol). Après immuno-absorption les feuilles ont été enlevées puis trempées dans la solution de lavage contenant le TRITON x 100 pendant 20 minutes. Les débris cellulaires encore adhérents ont été éliminés par des jets de la même solution produits par une seringue. Les feuilles ont alors été placées dans la solution fraîche jusqu'à ce qu'aucun débris ne soit plus visible. Les immuno-complexes ont alors été identifiés comme décrit par M. KOENEN et coll..

36 clones positifs réagissant avec les anticorps de lapin ont ainsi pu être isolés. Les colonies correspondantes ont alors été triées dans des conditions semblables, mais en mettant cette fois en oeuvre les sérums purifiés humains qui ont été rappelés plus haut.

Parmi ces clones, il en est un qui a réagi d'une façon particulièrement forte avec le sérum anti-humain. L'examen des produits de ces clones par électrophorèse sur gel d'acrylmaide au dodecylsulfate de sodium à permis de constater l'absence de la bande peptidique de 116 kilodaltons correspondant normalement à la β-galactosidase et la présence d'une nouvelle bande de 150 kilodaltons, bande qui a été isolée en quantités relativement importantes dans la fraction cytoplasmique de E. coli et non dans la membrane.

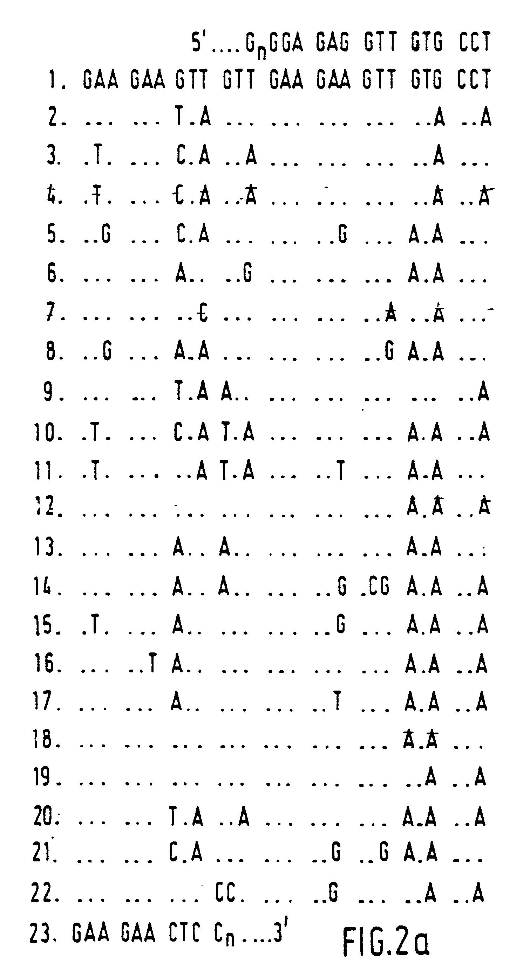

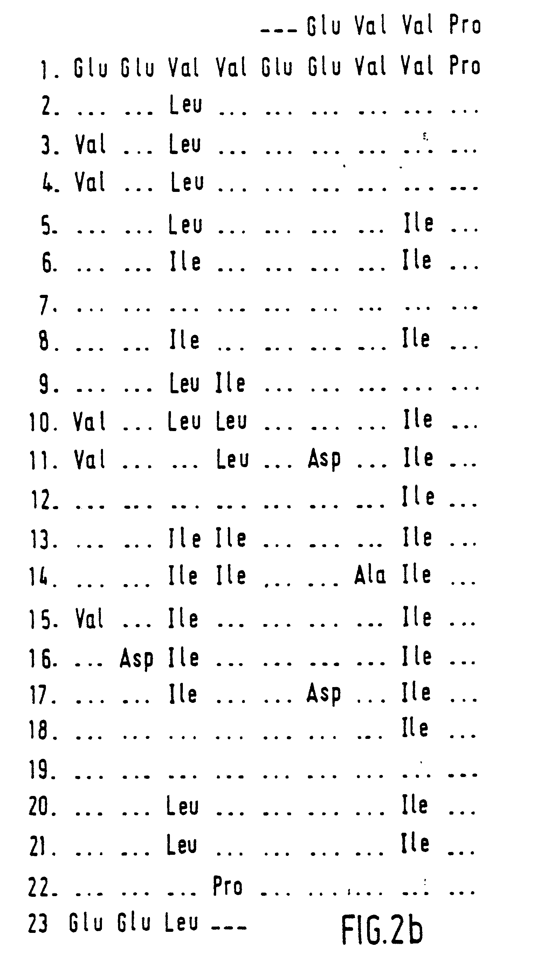

Ce résultat était en harmonie avec la taille de l'insérat d'environ 620 paires de bases qui avait été incorporé dans le vecteur. Cet insérat a été séquencé dans les deux directions selon la technique de MAXAM et GILBERT. Il a pu être déduit, en particulier de la structure des séquences d'extrémités de 620 paires de bases, que l'insérat originaire du génôme de Plasmodium falciparum contenait sur toute sa longueur des séquences répétitives de 27 paires de bases codant pour un polypeptide répétitif de 9 aminoacides: Glu Glu Leu Val Glu Glu Val Val Pro, si ce n'est que l'on a pu observer un certain nombre de variations dans ces séquences. En particulier on à pu constater que les acides aminés isoleucine, leucine et valine pouvaient mutuellement se remplacer. Les observations faites sur ces séquences tendent à suggérer l'existence d'une helice en α comportant des interruptions par les prolines. Des séquences d'ADN caractéristiques et les séquences peptidiques pour lesquelles elles codent resultent de la fig. 2.

Le polypeptide ainsi exprimé est donc caractéristique de ceux qui sont conformes à l'invention.

Le fait que la proteine chimère susdite est reconnue par les anticorps humains soutient l'hypothèse que le déterminant antigénique caractéristique est immunogène chez l'homme. Les techniques d'analyse de l'ADN génonique sur "buvard de Southern" (Southernblot) selon la technique décrite par SOUTHER E. (1975) J. Mol, Biol. 98, 503, révèlent, après digestion du fragment concerné par l'enzyme Hind III, une bande d'environ 20 à 25 kb. La technique au "buvard de Northern" (THOMAS P. F. (1980) Proc. Natl. Acad. Sci., 77, 5201) mise en oeuvre avec de l'ARN poly-A+ permet également l'identification d'un ARN messager ayant de 7 à 9 kb.

Le polypeptide contenant les séquences peptidiques répétitives conformes à l'invention presente des propriétés immunogènes. En particulier des anticorps formés contre la molécule chimère β-galactosidase-fragment (dans laquelle la β-galactosidase peut être considérée comme jouant un rôle de support) se sont révélés réagir avec un antigène associé aux globules rouges infectés par le P. falciparum aux stades tardifs, réaction révélée par immunofluorescence indirecte et produisant une image caractéristique. Cette même image d'immunofluorescence á été observée avec plusieurs souches de P. falciparum (Palo Alto, NF 54, It G2G1). Ceci permet d'envisager d'utiliser la séquence peptidique pour un diagnostic de P. falciparum par toutes les méthodes immmologiques connues de l'homme de l'art, telles que Elisa, Immunofluorescence, etc..

L'antigène de P. falciparum reconnu par les sérums a, dans des conditions expérimentales précises (Extraction de P. falciparum par du Triton à 1 % puis électrophorèse en gel de SDS-polyacrylamide à 6,5 %), un poids moléculaire de l'ordre de 250 000 daltons.

Les molécules selon l'invention peuvent donc, grâce à leur antigénicité, être utilisées comme réactifs, notamment pour la détection d'anticorps dirigés contre des protéines résultant de l'activité infectieuse des parasites du paludisme, notamment dans leur phase de développement érythrocytaire, et plus particulièrement contre des protéines qui s'associent aux membranes des globules infectés. Parmi ces protéines on mentionnera celles qui peuvent intervenir dans la fixation des globules rouges infectés aux cellules endothéliales des capillaires du cerveau, que l'on peut observer dans les formes cérébrales du paludisme. Ces manifestations peuvent être à l'origine de thromboses paludiques, souvent fatales pour le malade.

A l'inverse, les peptides ou oligopeptides selon l'invention peuvent encore être utilisées pour la production d'anticorps in vivo, par l'immunisation de l'animal, tel que le lapin, ces anticorps pouvant à leur tour être utilisés pour le diagnostic in vitro de l'apparition de protéines de membranes ou exposées à la surface des membranes des érythrocytes, à certains stades d'évolution de l'infection parasitaire.

L'invention ouvre enfin la voie à la mise au point de nouveaux principes de vaccinants contre ces phénomènes immunopathologiques particulièrement pernicieux. En particulier les résultats qui ont été évoqués permettent d'envisager l'utilisation des peptides ou oligopeptides selon l'invention, liés ou non de façon covalente à un support, en tant que principes protecteurs contre la fixation des érythrocytes infectés sur les parois des capillaires cérébraux. A ce titre les molécules selon l'invention peuvent constituer l'un des principes actifs des vaccins actifs contre le paludisme.

L'invention concerne donc également les compositions préparées sous forme de vaccins contenant soit le peptide selon l'invention, soit un oligomère de ce peptide, soit encore un conjugué de ce peptide ou oligomère avec une molécule porteuse, en association avec un véhicule approprié et, le cas échéant, avec d'autres principes actifs vaccinants contre le paludisme.

Des compositions pharmaceutiques avantageuses sont constituées par des solutions, suspensions ou liposomes injectables contenant une dose efficace d'au moins un produit selon l'invention. De préférence, ces solutions, suspensions ou liposomes sont réalisés dans une phase aqueuse stérilisée isotonique, de préférence saline ou glucosée.

L'invention concerne plus particulièrement de telles suspensions, solutions ou forme liposome qui sont aptes à être administrées par injections intradermiques, intramusculaires ou sous-cutanées, ou encore par scarifications.

Elle concerne également des compositions pharmaceutiques administrables par d'autre voies, notamment par voie orale ou rectale, ou encore soms forme d'aérosols destinés à venir en contact avec des muqueuses, notamment les muqueuses oculaires, nasales, pulmonaires ou vaginales.

En conséquence, elle concerne des compositions pharmaceutiques dans lesquelles l'un au moins des produits selon l'invention se trouve associé à des excipients pharmaceutiquement acceptables, solides ou liquides, adatpés à la constitution de formes orales, oculaires ou nasales, ou avec des excipients adaptés à la constitution des formes d'administration rectale, ou encore avec des excipients gélatineux pour l'administration vaginale. Elle concerne aussi des compositions liquides isotoniques contenant l'un au moins des conjugués selon l'invention, adaptées à l'administration sur les muqueuses, notamment oculaires ou nasales.

Avantageusement, les compositions vaccinales selon l'invention contiennent en outre un véhicule, tel que la polyvinyl-pyrrolidone, facilitant l'administration du vaccin. A la place de la polyvinyl-pyrrolidone, on peut utiliser tout autre type d'adjuvant au sens classique que l'on donnait autrefois à cette expression, c'est-à-dire d'une substance permettant l'absorption plus aisée d'un médicament ou facilitant son action dans l'organisme. A titre d'exemples d'autres adjuvants de ce dernier type, on mentionnera encore la carboxyméthyl-cellulose, les hydroxydes et phophates d'aluminium ou tous autres adjuvants de ce type, bien connus de l'homme de l'art. Enfin elles contiennent si besoin un adjuvant immunologique, notamment du type muramylpeptide.

Il est naturellement bien entendu que les peptides qui résultent de telles substitutions consistent en des équivalents des peptides plus particulièrement revendiqués, dès lors qu'eux-mêmes ou des oligomères ou conjugués formés à partir de ces peptides présentent des propriétés immunogènes semblables.

L'invention concerne également encore plus particulièrement les "protéines chimères" qui peuvent être obtenues par les techniques du génie génétique, ces protéines chimères pouvant contenir une ou plusieurs séquences peptidiques, notamment non-apeptidiques conformes à l'invention et incorporées ou rattachées à un fragment peptidique autre que la beta-galactosidase. Ce dernier fragment peptidique á de préférence un poids moléculaire suffisant pour renforcer l'immunogénicité des séquences peptidiques selon l'invention et n'interfère pas du point de vue immunologique avec la manifestation de l'immunogénicité recherchée.

Les séquences nucléotidiques correspondant aux séquences peptidiques sus-visées peuvent être utilisées selon des techniques connues d'hybridation moléculaire, comme agent de diagnostic de P. falciparum.

De même, les peptides codes par ces sequences nucléotidiques peuvent être utilisés pour la détection dans les sérums de patients d'aticorps dirigés contre la susdite protéine associée aux membranes des globules éventuellement infectées. A l'inverse, ces peptides peuvent également être utilisés pour la fabrication d'anticorps, notamment chez l'animal, utilisables à leur tour pour la détection in vitro d'une infection par des parasites paludiques, notamment par mise en contact avec les globules susceptibles d'être infectés dans des conditions permettant une éventuelle réaction immunologique.

## Revendications

1. Polypeptide formé, soit d'un monomère synthétique nonapeptidique répondant respectivement à l'une des formules indiquées ci-dessous, soit d'un oligomère de l'un ou de plusieurs des monomères dont les formules sont indiquées ci-dessous:

|       |     |     |     |     |     |     |     |     |     |
|-------|-----|-----|-----|-----|-----|-----|-----|-----|-----|
| 1.    | Glu | Glu | Val | Val | Glu | Glu | Val | Val | Pro |
| 2.    | ... | ... | Leu | ... | ... | ... | ... | ... | ... |
| 3.    | Val | ... | Leu | ... | ... | ... | ... | ... | ... |
| 5.    | ... | ... | Leu | ... | ... | ... | ... | Ile | ... |
| 6.    | ... | ... | Ile | ... | ... | ... | ... | Ile | ... |
| 9.    | ... | ... | Leu | Ile | ... | ... | ... | ... | ... |
| 10.   | Val | ... | Leu | Leu | ... | ... | ... | Ile | ... |
| 11.   | Val | ... | ... | Leu | ... | Asp | ... | Ile | ... |
| 13.   | ... | ... | Ile | Ile | ... | ... | ... | Ile | ... |
| 14.   | ... | ... | Ile | Ile | ... | ... | Ala | Ile | ... |
| 15.   | Val | ... | Ile | ... | ... | ... | ... | Ile | ... |
| 16.   | ... | Asp | Ile | ... | ... | ... | ... | Ile | ... |
| 17.   | ... | ... | Ile | ... | ... | Asp | ... | Ile | ... |
| 20.   | ... | ... | Leu | ... | ... | ... | ... | Ile | ... |
| 22.   | ... | ... | ... | Pro | ... | ... | ... | ... | ... |
| 23.   | Glu | Glu | Leut| ... | ... | ... | ... | ... | ... |

étant entendu que dans chacune des séquences nonapeptidiques ci-dessus les groupes des trois points respectivement situés à la verticale des résidus aminoacyle de la première des séquences nonapeptidiques susmentionnées sont identiques à ces aminoacyles et que, dans ces monomères:

- chacun des acides aminés isoleucine, leucine et valine peut, dans ces séquences, être remplacé par l'un des deux autres, et que
  - Glu peut être remplacé par Asp ou Gln,
  - Val peut être remplacé par Ala ou Gly,
  - Leu peut être remplacé par Ala.

2. Polypeptide selon la revendication 1, caractérisé en ce qu'il est constitué d'un peptide nonapeptidique.

3. Polypeptide selon la revendication 1, caractérisé en ce qu'il est constitué par un oligomère d'une ou plusieurs des sus-dites séquences, cet oligomère contenant de 2 à 25 unités monomère.

4. Polypeptide selon l'une quelconque des revendications 1 à 3, caractérisé en ce qu'il est fixé à un support macromoléculaire physiologiquement acceptable et non toxique, n'entraînant pas une modification des propriétés immunogéniques dudit polypeptide, telles que l'anatoxine tétanique, l'ovalbumine, des sérums albumines, des hémocyanines, ou des polylysines ou des poly (D-L-alanine)-poly(L-lysine) présentent en général un poids moléculaire supérieur à 20 000.

5. Protéine chimère contenant le polypeptide selon l'une quelconque des revendications 1 à 3 incorporé dans un fragment peptidique présentant un poids moléculaire suffisant pour renforcer l'immunogénicité dudit polypeptide et n'interférant pas du point de vue immunologique vec la manifestation de l'immunogénicité dudit polypeptide.

6. Composition immunogène caractérisée par l'association d'une molécule conforme à l'une quelconque des revendications 1 à 5, avec un véhicule pharmaceutique acceptable.

7. Composition de vaccin dirigée contre le paludisme, contenant entre autres principes immunogènes une molécule conforme à l'une quelconque des revendications 1 à 5.

8. Séquence de nucléotides codant pour une ou plusieurs des séquences peptidiques définies dans la revendication 1 ou la revendication 2.

9. Application des molécules conformes à l'une quelconque des revemdications 1 à 5 comme agents de diagnostic.

10. Application de la séquence de nucléotides selon la revendication 8 comme agent de diagnostic, par

7

hybridation moléculaire avec les acides nucléiques de P. falciparum infectant les cellules, notamment érythrocytes de l'hôte.

**Patentansprüche**

1. Polypeptid, dass entweder aus einem synthetischen, nonapeptidischen Monomer, das jeweils einer der unten aufgeführten Formeln entspricht, oder aus einem Oligomer von einem oder mehreren der Monomere, deren Formeln unten aufgeführt sind, gebildet ist

| | 1 | 2 | 3 | 4 | 5 | 6 | 7 | 8 | 9 |
|---|---|---|---|---|---|---|---|---|---|
| 1. | Glu | Glu | Val | Val | Glu | Glu | Val | Val | Pro |
| 2. | ... | ... | Leu | ... | ... | ... | ... | ... | ... |
| 3. | Val | ... | Leu | ... | ... | ... | ... | ... | ... |
| 5. | ... | ... | Leu | ... | ... | ... | ... | Ile | ... |
| 6. | ... | ... | Ile | ... | ... | ... | ... | Ile | ... |
| 9. | ... | ... | Leu | Ile | ... | ... | ... | ... | ... |
| 10. | Val | ... | Leu | Leu | ... | ... | ... | Ile | ... |
| 11. | Val | ... | ... | Leu | ... | Asp | ... | Ile | ... |
| 13. | ... | ... | Ile | Ile | ... | ... | ... | Ile | ... |
| 14. | ... | ... | Ile | Ile | ... | ... | Ala | Ile | ... |
| 15. | Val | ... | Ile | ... | ... | ... | ... | Ile | ... |
| 16. | ... | Asp | Ile | ... | ... | ... | ... | Ile | ... |
| 17. | ... | ... | Ile | ... | ... | Asp | ... | Ile | ... |
| 20. | ... | ... | Leu | ... | ... | ... | ... | Ile | ... |
| 22. | ... | ... | ... | Pro | ... | ... | ... | ... | ... |
| 23. | Glu | Glu | Leu | ... | ... | ... | ... | ... | ... |

wobei in jeder der aufgeführten nonapeptidischen Sequenzen die Gruppen der drei Punkte, die jeweils an der Vertikale der Aminoacylreste der ersten der obenerwähnten nonapeptidischen Sequenzen plaziert sind, mit diesen Aminoacylen identisch sind, und daß in diesen Monomeren - jede der Aminosäuren Isoleucin, Leucin und Valin in diesen Sequenzen untereinander ausgetauscht werden kann, und daß

- Glu durch Asp oder Gln ersetzt werden kann,
- Val durch Ala oder Gly ersetzt werden kann,
- Leu durch Ala ersetzt werden kann.

2. Polypeptid nach Anspruch 1, dadurch gekennzeichnet, daß es aus einem nonapeptidischen Peptid gebildet ist.

3. Polypeptid nach Anspruch 1, dadurch gekennzeichnet, daß es aus einem Oligomer einer oder mehrerer der obengenannten Sequenzen gebildet ist, wobei das Oligomer 2 bis 25 Monomereinheiten enthält.

4. Polypeptid nach einem der Ansprüche 1 bis 3, dadurch gekennzeichnet, daß es an einen makromolekularen Träger, der physiologisch verträglich und nicht toxisch ist, gebunden ist und keine Veränderungen der immunogenen Eigenschaften des besagten Polypeptids nach sich zieht, wie Tetanusanatoxin, Ovalbumin, Albuminseren, Hämocyanine, Polylysine oder Poly(D-L-Alanin)-poly(L-Lysine), die im allgemeinen ein Molekulargewicht von höher als 20000 aufweisen.

5. Chimäres Protein, das das Polypeptid nach einem der Ansprüche 1 bis 3 enthält und einem peptidischen Fragment zugesetzt wird, das ein Molekulargewicht aufweist, das ausreicht, um die Immunogenizität des besagten Polypeptids zu verstärken, und, vom immunologischen Standpunkt aus, das Vorhandensein der Immunogenizität des besagten Polypeptids nicht beeinträchtigt.

6. Immunogene Zusammensetzung, gekennzeichnet durch die Verbindung eines Moleküls nach einem der Ansprüche 1 bis 5, mit einem pharmazeutisch verträglichen Träger.

7. Impfstoffzusammensetzung gegen Malaria, die neben anderen immunogenen Bestandteilen ein Molekül nach einem der Ansprüche 1 bis 5 enthält.

8. Nukleotidsequenz, die eine oder mehrere der in Anspruch 1 oder 2 definierten Peptidsequenzen kodiert.

9. Anwendung der Moleküle nach einem der Ansprüche 1 bis 5 als Diagnostizierungsmittel.

10. Anwendung der Nukleotidsequenz nach Anspruch 8 als Diagnostizierungsmittel, durch molekulare Hybridisierung mit den Nukleinsäuren von P. falciparum, das die Zellen, insbesondere die Erythrozyten, eines Wirts infiziert.

## Claims

1. Polypetide formed either of a nonapeptidic synthetic monomer having respectively one of the formulae indicated below, or of an oligomer of one or several of say monomers whose formulae are indicated below:

| | | | | | | | | | |
|---|---|---|---|---|---|---|---|---|---|
| 1. | Glu | Glu | Val | Val | Glu | Glu | Val | Val | Pro |
| 2. | ... | ... | Leu | ... | ... | ... | ... | ... | ... |
| 3. | Val | ... | Leu | ... | ... | ... | ... | ... | ... |
| 5. | ... | ... | Leu | ... | ... | ... | ... | Ile | ... |
| 6. | ... | ... | Ile | ... | ... | ... | ... | Ile | ... |
| 9. | ... | ... | Leu | Ile | ... | ... | ... | ... | ... |
| 10. | Val | ... | Leu | Leu | ... | ... | ... | Ile | ... |
| 11. | Val | ... | ... | Leu | ... | Asp | ... | Ile | ... |
| 13. | ... | ... | Ile | Ile | ... | ... | ... | Ile | ... |
| 14. | ... | ... | Ile | Ile | ... | ... | Ala | Ile | ... |
| 15. | Val | ... | Ile | ... | ... | ... | ... | Ile | ... |
| 16. | ... | Asp | Ile | ... | ... | ... | ... | Ile | ... |
| 17. | ... | ... | Ile | ... | ... | Asp | ... | Ile | ... |
| 20. | ... | ... | Leu | ... | ... | ... | ... | Ile | ... |
| 22. | ... | ... | ... | Pro | ... | ... | ... | ... | ... |
| 23. | Glu | Glu | Leu | ... | ... | ... | ... | ... | ... |

it being understood that in each of said nonapeptidic sequences, the three groups respectively located at the vertical of the aminoacyl residues of the first of said nonapeptidic sequences are identical to these aminoacyls and that, in said monomers:

- each of the isoleucine, leucine and valine aminoacid residues may, in said sequences, be replaced by one of the two others, and that
- Glu may be replaced by Asp or Gln,
- Val may be replaced by Ala or Gly,
- Leu may be replaced by Ala.

2. Polypeptide according to claim 1, characterized in that it is formed of a nonapeptidic peptide.

3. Polypeptide according to claim 1, characterized in that it is formed of an oligomer of one or several of said sequences, said oligomer containing from 2 to 25 monomer units.

4. Polypeptide according to any one of claims to 3, characterized in that it is bound to a physiologically acceptable and non toxic macromolecular support which does not provide for a modification of the immunogenic properties of said polypeptide, such tetanus toxoid, ovalbumin, serum albumins, hemocyanines, or polylysines or poly(D-L-alanine)poly(L-lysine) which generally presents a molecular weight above 20 000.

5. Chimeric protein containing one polypeptide according to any one of claims 1 to 3, incorporated into a peptide fragment presenting a molecular weight sufficient to reinforce the immunogenicity of say polypeptide and which does not interfere from the immunological point of view with the manifestation of the immunogenicity of said polypeptide.

6. Immunogenic composition characterized by the association of a molecule in conformity with anyone of claims 1 to 5 with a pharmaceutically acceptable vehicle.

7. Vaccine composition directed against paludism, which contains among other immunogenic principles, a molecule in accordance with anyone of claims 1 to 5.

8. Nucleotide sequence coding for one or several of the peptidic sequences defined in claim 1 or claim 2.

9. Use of the molecules according to any one of claims 1 to 5 as diagnostic agents.

10. Use of the nucleotide sequence according to claim 8 as diagnostic agents through molecular hybridization with nucleic acids of P. falciparum infecting cells, particularly erythrocytes of the host.

# FIG.1.

```
         5'....GₙGGA GAG GTT GTG CCT
 1. GAA GAA GTT GTT GAA GAA GTT GTG CCT
 2. ... ... T.A ... ... ... ... ..A ..A
 3. .T. ... C.A ..A ... ... ... ..A ...
 4. .T. ... C.A ..A ... ... ... ..A ..A
 5. ..G ... C.A ... ... ..G ... A.A ...
 6. ... ... A.. ..G ... ... ... A.A ...
 7. ... ... ..C ... ... ... ..A ..A ...
 8. ..G ... A.A ... ... ... ..G A.A ...
 9. ... ... T.A A.. ... ... ... ... ..A
10. .T. ... C.A T.A ... ... ... A.A ..A
11. .T. ... ..A T.A ... ..T ... A.A ...
12. ... ... ... ... ... ... ... A.A ..A
13. ... ... A.. A.. ... ... ... A.A ...
14. ... ... A.. A.. ... ..G .CG A.A ..A
15. .T. ... A.. ... ... ..G ... A.A ..A
16. ... ..T A.. ... ... ... ... A.A ..A
17. ... ... A.. ... ... ..T ... A.A ..A
18. ... ... ... ... ... ... ... A.A ...
19. ... ... ... ... ... ... ... ..A ..A
20. ... ... T.A ..A ... ... ... A.A ..A
21. ... ... C.A ... ... ..G ..G A.A ...
22. ... ... ... CC. ... ..G ... ..A ..A
23. GAA GAA CTC Cₙ....3'        FIG.2a
```

3

```
                          --- Glu Val Val Pro
 1. Glu Glu Val Val Glu Glu Val Val Pro
 2. ... ... Leu ... ... ... ... ...
 3. Val ... Leu ... ... ... ...  .. ...
 4. Val ... Leu ... ... ... ... ...
 5. ... ... Leu ... ... ... ... Ile ...
 6. ... ... Ile ... ... ... ... Ile ...
 7. ... ... ... ... ... ... ... ...
 8. ... ... Ile ... ... ... ... Ile ...
 9. ... ... Leu Ile ... ... ... ...
10. Val ... Leu Leu ... ... ... Ile ...
11. Val ... ... Leu ... Asp ... Ile ...
12. ... ... ... ... ... ... ... Ile ...
13. ... ... Ile Ile ... ... ... Ile ...
14. ... ... Ile Ile ... ... Ala Ile ...
15. Val ... Ile ... ... ... ... Ile ...
16. ... Asp Ile ... ... ... ... Ile ...
17. ... ... Ile ... ... Asp ... Ile ...
18. ... ... ... ... ... ... ... Ile ...
19. ... ... ... ... ... ... ... ...
20. ... ... Leu ... ... ... ... Ile ...
21. ... ... Leu ... ... ... ... Ile ...
22. ... ... ... Pro ... ... ... ...
23  Glu Glu Leu ---
```

FIG.2b